# EUROPEAN PATENT APPLICATION

(11) **EP 1 916 012 A1**
(43) Date of publication of application: **30.04.2008**
(21) Application number: 06782781.6
(22) Date of filing: 17.08.2006
(51) Int. Cl.: A61N 1/30, A61M 37/00

(54) **METHOD AND DEVICE FOR MANAGING USE CONDITION OF MEDICINE, AND MEDICINE INJECTION DEVICE**

(30) Priority: 18.08.2005 JP 2005237755
(71) Applicant: TTI Ellebeau, Inc., Tokyo 150-0022 (JP)
(72) Inventor: NAKAYAMA, Mizuo, Tokyo 150-0022 (JP); MATSUMURA, Takehiko, Tokyo 150-0022 (JP); AKIYAMA, Hidero, Tokyo 150-0022 (JP); MATSUMURA, Akihiko, Tokyo 150-0022 (JP)
(74) Representative: Carstens, Dirk Wilhelm
(86) International application number: PCT/JP2006/316168
(87) International publication number: WO 2007/020975

(57) **Abstract**

A drug-injecting device driven by a drive signal is provided with a means for identifying an ID of a patient. The drug-inj ecting device 120 (an iontophoresis device 10, 50, or 60) is driven by the drive signal made valid after the ID of the patient to whom the drug is to be administered is authenticated. The use condition of a drug can be managed by a server 130 on the basis of the actual information about the driving of the drug-injecting device 120. A prescribed drug can be confirmed whether used property or not.

## Description

### [Technical Field]

The present invention relates to a method and an apparatus for managing the use condition of a drug, and a drug-inj ecting device. In particular, the present invention relates to a method and an apparatus for managing the use condition of a drug each of which is suitably used in managing the use condition of a drug to be administered by means of a drug-injecting device such as an iontophoresis device driven by a driving signal and with each of which the use condition of the drug can be managed on the basis of an actual use condition, and a drug-injecting device suitably used for the method and apparatus.

### [Background Art]

A patient has conventionally decided at his or her free will whether he or she actually takes a drug prescribed by a doctor, and it has been impossible to confirm whether the patient took the drug prescribed by the doctor as indicated by the doctor. Therefore, at present, half or more of the drugs prescribed by doctors and sent to patients have been disposed of, and become considerable waste. The waste is a large problem in achieving a reduction in medical expenses. This problem cannot be avoided as long as a patient takes a drug at his or her free will from his or her mouth or the like.

On the other hand, iontophoresis has been proposed as a method of permeating a drug into an organism through the skin or mucosa of the organism. A device for conducting the iontophoresis (referred to as "iontophoresis device") includes a working electrode assembly having a drug solution holding portion holding a drug solution and a non-working electrode assembly as a counter electrode of the working electrode assembly. A voltage having the same conductivity type as that of a drug ion in the drug solution holding portion is applied to the working electrode assembly in a state where the drug solution is brought into contact with the skin or mucosa of an organism to electrically drive the drug ion, whereby the drug ion is transferred into the organism through the skin or mucosa.

For example, WO 03/037425A1 describes an iontophoresis device capable of administering an ionic drug stably for a long time period and at a high transport number. In the iontophoresis device, each of the constituent elements, that is, a working electrode assembly and a non-working electrode assembly is constituted in a film state. The working electrode assembly is provided with ion exchange membranes, which are different from each other in ion selectivity, for selecting an ion having the same polarity as that of a charged ion of the ionic drug and an ion opposite in polarity to that of the charged ion. In addition, the non-working electrode assembly is provided with at least an ion exchange membrane for selecting an ion opposite in polarity to that of the charged ion of the ionic drug.

However, it has been conventionally considered to be impossible to manage the use condition of a drug on the basis of its actual use condition.

### [Disclosure of the Invention]

The present invention has been made with a view to solving the conventional problems, and a first obj ect of the present invention is to manage the use condition of a drug to be administered to a patient on the basis of the actual use condition of the drug.

A second object of the present invention is to provide a drug-injecting device suitable for the above-mentioned management of the use condition of a drug.

Above objects can be solved by following examples.

According to the invention of claim 1, management of a use condition of drug to be administered using a drug injecting device driven by a drive signal is performed on the basis of actual drive information of the drug injecting device, to thereby achieve the first object of the present invention.

According to the invention of claim 2, the drug injecting device makes a drive signal valid after an ID of a patient to whom the drug is to be administered is authenticated.

According to the invention of claim 3, the ID of the patient is in advance placed on the patient.

According to the invention of claim 4, the ID of the patient is in advance implanted in the patient.

According to the invention of claim 5, the drug injecting device includes an iontophoresis device, which includes a working electrode assembly having a drug solution holding portion holding a drug solution and a non-working electrode assembly as a counter electrode of the working electrode assembly, and a voltage having the same conductivity type as that of a drug ion in the drug solution holding portion is applied to the working electrode assembly in a state where the drug solution is brought into contact with the skin or mucosa of an organism to electrically drive the drug ion, whereby the drug ion is transferred into the organism through the skin or mucosa.

According to the invention of claim 6, there is provided a device for managing a use condition of drug characterized by including:
a drug injecting device driven by a drive signal, which can output actual drive information; and
a server for managing a use of a drug to be administered using the drug injecting device on the basis of actual drive information of the drug injecting device.

According to the present invention of claim 7, the server manages on a patient basis a kind, an amount, and a timing of a drug administered to a patient.

According to the present invention of claim 8, information on a drug administered by the drug injecting device is stored in the drug injecting device and is later read into the server.

According to the present invention of claim 9, information on a drug administered by the drug injecting device is sent to the server by radio communication.

According to the present invention of claim 10, a drug-injecting device driven by a drive signal, comprises a means for identifying an ID of a patient, whereby the drive signal is made valid after the ID of the patient to whom a drug is to be administered is authenticated, to thereby achieve the second object of the present invention.

According to the invention according to claim 1, the use condition of a drug to be administered by means of a drug-injecting device is managed on the basis of the actual information about the driving of the drug-injecting device. As a result, it becomes possible to confirm whether a drug prescribed by a doctor is properly used for a patient. Therefore, the doctor can adequately understand the kind and amount of the drug used and the action of the drug on the patient. In addition, the disposal of part of the drug prescribed by the doctor without being administered to the patient is eliminated, so the waste of medical expenses is eliminated.

In addition, according to the invention of claim 2, the drug-injecting device is adapted to validate a driving signal after ithas identified the IDof a patient towhomadrug is tobe administered. As a result, the drug can be administered to only a patient as an object of prescription.

According to the invention of claim 3, the ID of the patient is mounted on the patient in advance. As a result, erroneous input of the patient's ID can be prevented.

According to the invention of claim 4, the ID of the patient is implanted in the body of the patient in advance. As a result, erroneous mounting of the patient's ID on the patient can also be prevented.

According to the invention of claim 5, the drug-injecting device is an iontophoresis device. As a result, the use condition of a drug can be adequately managed.

According to the invention of claim 6, administration history can be managed by means of information accumulated in a server.

According to the invention of claim 7, the kind and amount of a drug administered to a patient and the time at which the drug was administered to the patient can be managed for each patient.

According to the invention of claim 8, information about the administration of a drug by a drug-injecting device can be collectively input to a server with ease.

According to the invention of claim 9, information about the administration of a drug by a drug-injecting device can be taken in a server in real time.

According to the invention of claim 10, there can be provided a drug-injecting device suitable for the management of the use condition of a drug because a driving signal is validated after the ID of a patient to whom a drug is to be administered has been identified.

### [Brief Description of the Drawings]

[Fig. 1] A block diagram showing the entire constitution of an embodiment of an apparatus for managing the use condition of a drug according to an embodiment of the present invention.
[Fig. 2] A perspective view showing a wrist band mounted with an IC chip to be used in the embodiment.
[Fig. 3] Aplan view showing a first example of an iontophoresis device as a drug-injecting device to be used in the embodiment.
[Fig. 4] An enlarged sectional view taken along the line IV-IV of Fig. 3.
[Fig. 5] An enlarged sectional view taken along the line V-V of Fig. 3.

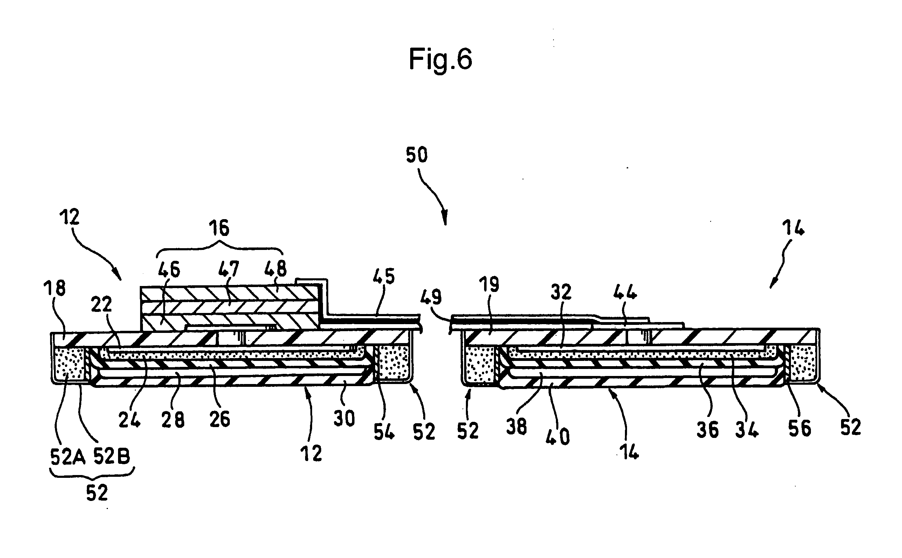

[Fig. 6] A sectional view showing the main portion of a second example of an iontophoresis device.

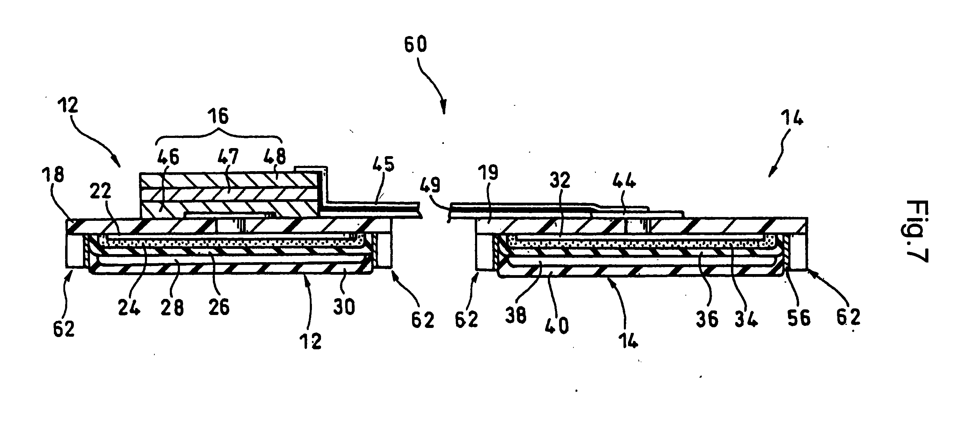

[Fig. 7] A sectional view showing the main portion of a third example of an iontophoresis device.
[Fig. 8] A perspective view showing an example of a stick having the drug-injecting device mounted on its tip.
[Fig. 9] A perspective view showing an example of a wrist band having the drug-injecting device mounted.
[Fig. 10] A perspective view showing an example of a stand having the drug-injecting device mounted on its tip.
[Fig. 11] A flow chart showing the operation of the embodiment.

### [Best Mode for carrying out the Invention]

Hereinafter, an embodiment of the present invention will be described in detail.

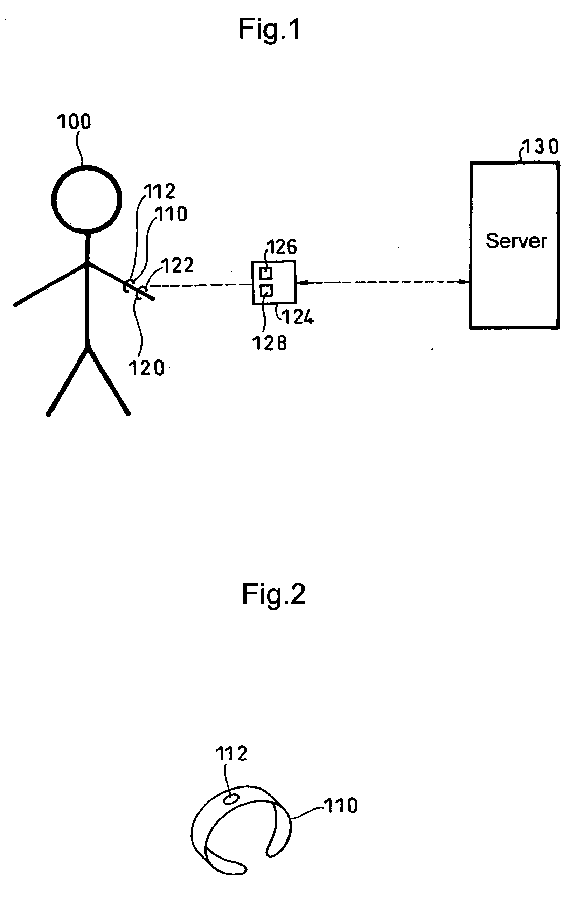

As shown in Fig. 1, a first embodiment of the present invention includes: a drug-injecting device 120 whose switch 122 can be turned on by a command from a wired or wireless remote controller (hereinafter, remote controller) 124 after the ID of a patient to whom a drug is to be administered has been identified by means of, for example, a wireless IC tag or IC chip 112 implanted in a wrist band 110 mounted on a patient 100; and a server 130 for managing the use condition of a drug to be administered by means of the drug-injecting device 120 on the basis of the actual information about the driving of the drug-injecting device 120.

As shown in Fig. 2, the IC chip 112 can be mounted on the patient 100 by being implanted in, for example, the wrist band 110 or a bracelet or by being implanted directly into the body of the patient 100. The erroneous description of information in the IC chip 112 can be prevented by constituting the chip in such a manner that the chip has an external electric power source like Suica (registered trademark) or FeliCa (registered trademark).

A patient's ID stored in the chip 122 can include only symbols for identifying the patient while the IC chip 112 is extremely reduced in size so that it can be implanted in the body of the patient or is adapted to be writable or rewritable by a reader/writer so as to include a contraindicated drug, the name of a disease, and administration history (the kind (name) of a drug, an administration time (year, month, day, hour, minute), and the amount of the drug to be administered) for each patient.

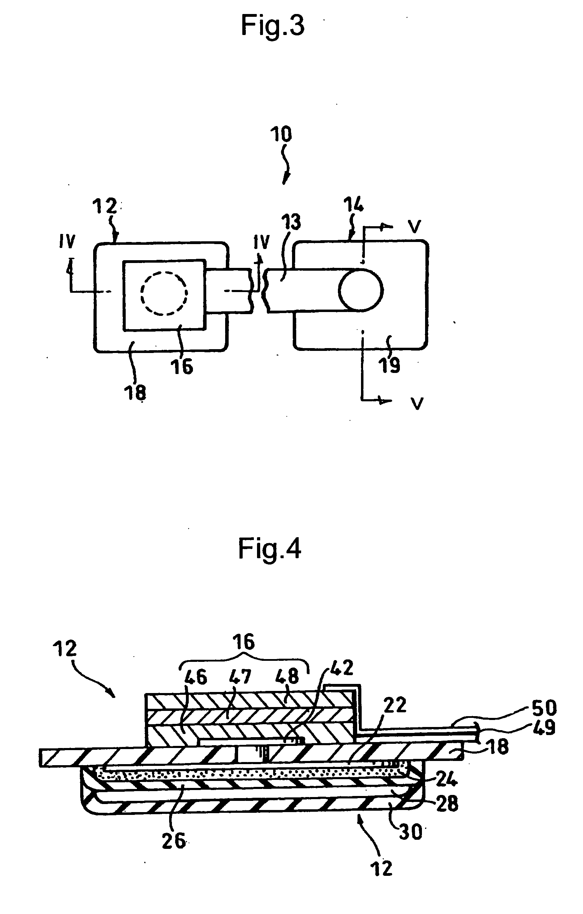

An iontophoresis device 10 shown in each of Figs. 3 to 5 can be used as the drug-injecting device 120.

An iontophoresis device 10 according to a first example of the best mode is constituted by a working electrode assembly 12 and a non-working electrode assembly 14 each used for administering an ionic drug and a DC electric power source 16 connected to the electrode assemblies 12 and 14 with opposite polarities.

The working electrode assembly 12 is constituted by laminating a working electrode 22, an electrolyte solution holding portion 24, a second ion exchange membrane 26, a drug solution holding portion 28, and a first ion exchange membrane 30 in this order from the side of a base sheet 18 on one surface (the lower surface in Fig. 4) of the base sheet 18.

The working electrode 22 is desirably constituted by a conductive paint applied to the one surface of the base sheet 18 and blended with a nonmetal conductive filler such as a carbon paste. The working electrode 22 can be constituted by a copper plate or a metal thin film, but a metal eluted from the plate or the thin film may transfer to an organism upon administration of a drug. Therefore, the working electrode 22 is preferably nonmetallic.

The electrolyte solution holding portion 24 is constituted by, for example, an electrolytic paint applied to the working electrode 22. The electrolytic paint is a paint containing an electrolyte, and an electrolyte that is oxidized or reduced more easily than the electrolytic reaction of water (oxidation on a positive electrode and reduction on a negative electrode) is particularly preferably used. Examples of such electrolyte include: medical agents such as ascorbic acid (vitamin C) and sodium ascorbate; and organic acids such as lactic acid, oxalic acid, malic acid, succinic acid, and fumaric acid and/or salts thereof. The use of such electrolyte can suppress the generation of an oxygen gas or a hydrogen gas. In addition, blending a plurality of kinds of electrolytes serving as a combination of buffer electrolyte solutions when dissolved in a solvent can suppress a change in pH during energization.

The electrolytic paint is blended with a hydrophilic polymer such as polyvinyl alcohol, polyacrylic acid, polyacrylamide, or polyethylene glycol in order to improve the application property and film-forming property of the paint, and is blended with an appropriate amount of solvent such as water, ethanol, or propanol for adjusting the viscosity of the electrolytic paint. The paint may be blended with an appropriate additional component such as a thickener, a thixotropic agent, a defoaming agent, a pigment, a flavor, or a coloring agent.

The second ion exchange membrane 26 is composed of an ion exchange resin into which an ion exchange group using, as a counter ion, an ion having a conductivity type opposite to that of a drug ion in the drug solution holding portion 28 to be described later is introduced. In the case where a drug whose drug component dissociates to plus drug ions is used in the drug solution holding portion 28, the membrane is blended with an anion exchange resin. On the other hand, in the case where a drug whose drug component dissociates to minus drug ions is used, the membrane is blended with a cation exchange resin.

The drug solution holding portion 28 is composed of a drug paint applied to the second ion exchange membrane 26. The paint is a paint containing a drug (including a precursor for the drug) whose drug component dissociates to plus or minus ions (drug ions) as a result of, for example, dissolution into a solvent such as water. Examples of a drug whose drug component dissociates to plus ions include lidocaine hydrochloride as an anesthetic drug and morphine hydrochloride as an anesthetic drug. Examples of a drug whose drug component dissociates to minus ions include ascorbic acid as a vitamin agent.

The first ion exchange membrane 30 is formed of a first ion exchange paint applied to the drug solution holding portion 28. The first ion exchange paint is a paint containing an ion exchange resin into which an ion exchange group using, as a counter ion, an ion having the same conductivity type as that of the drug ion in the drug solution holding portion 28 is introduced. In the case where a drug whose drug component dissociates to plus/minus drug ions is used in the drug solution holding portion 28, the paint is blended with an anion/cation exchange resin.

An ion exchange resin obtained by introducing a cation exchange group (an exchange group using a cation as a counter ion) such as a sulfonic group, a carboxylic group, or a phosphoric group into a polymer having a three-dimensional network structure such as a hydrocarbon-based resin (for example, a polystyrene resin or an acrylic resin) or a fluorine-based resin having a perfluorocarbon skeleton can be used as the cation exchange resin without any limitation.

An ion exchange resin obtained by introducing an anion exchange group (an exchange group using an anion as a counter ion) such as a primary amino group, a secondary amino group, a tertiary amino group, a quaternary ammonium group, a pyridyl group, an imidazole group, a quaternary pyridinium group, or a quaternary imidazolium group into a polymer having a three-dimensional network structure similar to that in the case of the cation exchange resin can be used as the anion exchange resin without any limitation.

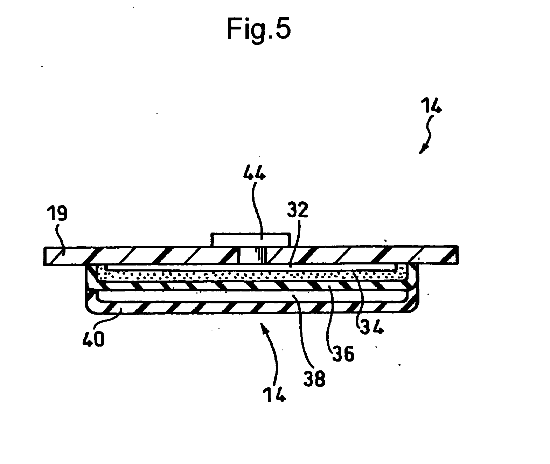

Fig. 5 is a partial enlarged view showing that the non-working electrode assembly 14 is constituted by laminating a non-working electrode 32, a second electrolyte solution holding portion 34, a third ion exchange membrane 36, a third electrolyte solution holding portion 38, and a fourth ion exchange membrane 40 in this order on one surface of a non-working base sheet 19 similar to the base sheet 18.

The non-working electrode 32 has the same constitution as that of the working electrode 22 in the cell-type working electrode assembly 12. In addition, the second electrolyte solution holding portion 34 is composed of the same electrolyte paint, which is applied to the non-working electrode 32, as that of the electrolyte solution holding portion 24. The third electrolyte solution holding portion 38 is composed of the same electrolyte paint, which is applied to the third ion exchange membrane 36, as that of the electrolyte solution holding portion 24.

Furthermore, the third ion exchange membrane 36 is composed of the same ion exchange resin as that present in the first ion exchange paint of which the first ion exchange membrane 30 is formed, and functions as an ion exchange membrane similar to the first ion exchange membrane 30.

The fourth ion exchange membrane 40 is formed of an ion exchange paint applied to the third electrolyte solution holding portion 38 and containing the ion exchange resin constituting the second ion exchange membrane 26. The fourth ion exchange membrane 40 functions as an ion exchange membrane similar to the second ion exchange membrane 26.

Aworking electrode terminal 42 is arranged on the other surface of the base sheet 18, and conduction is established between the working electrode terminal 42 and the working electrode 22 of the working electrode assembly 12 through a through-hole arranged on the base sheet 18.

Similarly, a non-working electrode terminal 44 is arranged on the other surface of the base sheet 19, and conduction is established between the non-working electrode terminal 44 and the non-working electrode 32 of the non-working electrode assembly 14 through a through-hole formed on the base sheet 19.

The DC electric power source 16 is placed between the working electrode terminal 42 and the non-working electrode terminal 44. The DC electric power source 16 is a cell-type battery, and is constituted by a first active electrode layer 46, a separator layer 47, and a second active electrode layer 48 laminated sequentially on one surface of the sheet-like pad 18 by means of an application method such as printing. The first active electrode layer 46 of the DC electric power source 16 and the working electrode terminal 42 are directly connected to each other, and the second active electrode layer 48 and the non-working electrode terminal 44 are connected to each other with a coating film (a non-working conductive layer) 45 of a conductive paint formed through an insulating paste layer 49.

Reference numeral 13 in Fig. 3 denotes a coupling belt for coupling the working electrode assembly 12 and the non-working electrode assembly 14. The coating film 45 is applied also to the coupling belt 13 and continues up to the non-working electrode terminal 44.

A thin battery constituting the DC electric power source 16 used can be one disclosed in, for example, JP 11-067236 A, US 2004/0185667 A1, or USP 6,855, 441, and the structure of the DC electric power source 16 is not limited to that disclosed in this mode.

In each of the above embodiments, the working electrode assembly 12 includes an electrolyte solution holding portion and two ion exchange membranes as well as a drug solution holding portion, and the non-working electrode assembly 14 includes two electrolyte solution holding portions and an ion exchange membrane. However, the present invention is not limited thereto. A drug solution holding portion may be constituted so as to directly contact with a working electrode.

In addition, a part of each of the electrolyte solution holding portions, the drug solution holding portion, and the ion exchange membranes constituting the working electrode assembly and the non-working electrode assembly except a part constituted by a temperature responsive gel is constituted by a coating film. However, the present invention is not limited thereto. Membranes maybe stuck to each other.

Furthermore, a drug-injecting device except an iontophoresis device driven by a driving signal, the drug-injecting device injecting a drug solution from, for example, a microneedle by means of a pump, can also be used as the drug-injecting device 120.

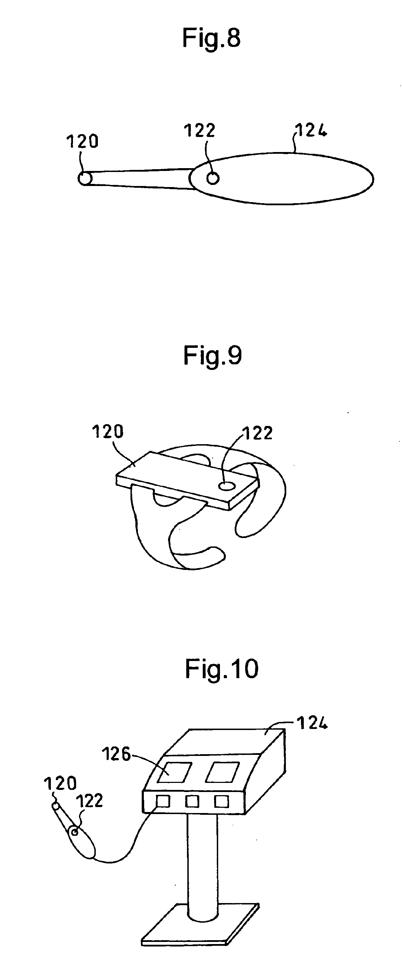

The drug-injecting device 120 may be of any one of a stick type shown in Fig. 8, a band type shown in Fig. 9, and a stand type shown in Fig. 10.

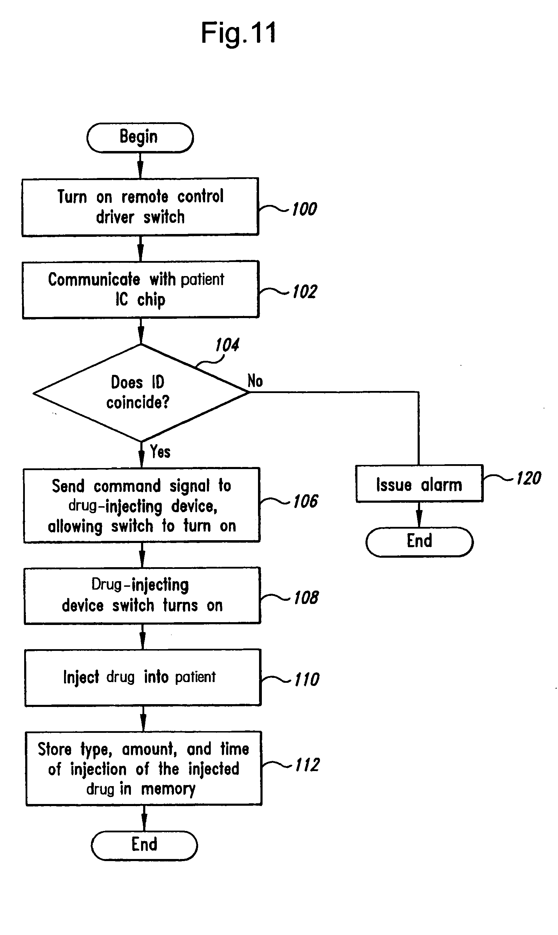

Hereinafter, an operation will be described with reference to Fig. 11.

When a driver switch 126 is turned on in a step 100, the remote controller 124 communicates with the IC chip 112 of the patient 100 in a step 102 to identify the ID of the patient. Only when the ID is judged to coincide with that of a patient as an object to whom an administration instruction is given in a step 104, a command signal is sent to the drug-injecting device 120 with or without a wire in a step 106 to enable the switch 122 of the drug-injecting device 120 to be turned on so that a drug can be injected into the patient 100. Then, when the switch 122 is actually turned on in a step 108, the drug is injected into the patient 110 in a step 110, and, at the same time, the kind and amount of the injected drug and the time at which the drug was injected are stored in a memory 128 rewritable with a built-in reader/writer in a step 112. Information stored in the memory 128 is thereafter collectively read in the server 130.

The memory 128 stores, for example, the name, amount, effective date, and manufacturer of a drug loaded into the drug-injecting device 120, and the ID of a patient to whom the drug is to be administered.

On the other hand, when the ID does not coincide with that of the patient as an object to whom an administration instruction is given in the step 104, an alarm is issued in a step 120.

In each of the above embodiments, information about the administration of a drug is stored in the memory 128 of the drug-injecting device 120, and is thereafter read in the server 130. Alternatively, each of the remote controller 124 and server 130 of the drug-injecting device 120 may be provided with a wireless device, or a portable phone may be used to send information about the administration of a drug to the server 130 in real time.

In each of the above embodiments, a patient's ID is stored in the IC chip 112. However, a method of storing the patient's ID is not limited thereto. For example, the following procedure may be adopted: a tape on which a one-dimensional or two-dimensional barcode is printed is stuck to the patient, and the drug-injecting device 120 or the remoter controller 124 is provided with a one-dimensional or two-dimensional barcode reader so that the barcode is read.

### [Industrial Applicability]

The present invention is suitable for use to manage the use condition of a drug to be administered by means of a drug-injecting device such as an iontophoresis device. The use condition of the drug can be managed on the basis of an actual use condition.

## Claims

1. A method of managing a drug use **characterized by** comprising managing a use of a drug to be administered using a drug injecting device driven by a drive signal on the basis of actual drive information of the drug injecting device.

2. The method of managing a drug use according to claim 1, **characterized in that** the drug injecting device makes a drive signal valid after an ID of a patient to whom the drug is to be administered is authenticated.

3. The method of managing a drug use according to claim 2, **characterized in that** the ID of the patient is in advance placed on the patient.

4. The method of managing a drug use according to claim 3, **characterized in that** the ID of the patient's body is in advance implanted in the patient.

5. The method of managing a drug use according to claim 1 or 2, **characterized in that** the drug injecting device comprises an iontophoresis device, which includes a working electrode assembly having a drug solution holding portion holding a drug solution and a non-working electrode assembly as a counter electrode of the working electrode assembly, and a voltage having the same conductivity type as that of a drug ion in the drug solution holding portion is applied to the working electrode assembly in a state where the drug solution is brought into contact with the skin or mucosa of an organism to electrically drive the drug ion, whereby the drug ion is transferred into the organism through the skin or mucosa.

6. A device for managing a drug use **characterized by** comprising:
a drug injecting device driven by a drive signal, which can output actual drive information; and
a server for managing a use of a drug to be administered using the drug injecting device on the basis of actual drive information of the drug injecting device.

7. The device for managing a drug use according to claim 6, **characterized in that** the server manages on a patient basis a kind, an amount, and a timing of a drug administered to a patient.

8. The device for managing a drug use according to claim 6 or 7, **characterized in that** information on a drug administered by the drug injecting device is stored in the drug injecting device and is later read into the server.

9. The device for managing a drug use according to claim 6 or 7, **characterized in that** information on a drug administered by the drug injecting device is sent to the server by radio communication.

10. A drug injecting device driven by a drive signal, comprising
a means for identifying an ID of a patient,
whereby the drive signal is made valid after the ID of the patient to whom a drug is to be administered is authenticated.
